# EUROPÄISCHE PATENTSCHRIFT

(11) **EP 4 190 228 B1**
(45) Veröffentlichungstag und Bekanntmachung des Hinweises auf die Patenterteilung: **25.03.2026**
(21) Anmeldenummer: 21212316.0
(22) Anmeldetag: 03.12.2021
(51) Int. Cl.: A61B 5/0537, A61B 5/0538, A61B 18/12, A61B 18/14

(54) **EINRICHTUNG ZUR GEWEBEIDENTIFIKATION**
DEVICE FOR TISSUE IDENTIFICATION
DISPOSITIF D'IDENTIFICATION DES TISSUS

(43) Veröffentlichungstag der Anmeldung: 07.06.2023
(73) Patentinhaber: Erbe Elektromedizin GmbH, 72072 Tübingen (DE)
(72) Erfinder: DAMMEIER, Sascha, 72070 Tuebingen (DE); NEUGEBAUER, Alexander, 72116 Moessingen (DE)
(74) Vertreter: Rüger Abel Patentanwälte PartGmbB

(56) Entgegenhaltungen:
- EP-A2- 2 491 882
- EP-B1- 2 659 846
- BASTIAN HILLEBRAND ET AL: "Tissue differentiation using optical emission spectroscopy for gastric mucosal devitalisation", JOURNAL OF PHYSICS D: APPLIED PHYSICS, INSTITUTE OF PHYSICS PUBLISHING, BRISTOL, GB, vol. 54, no. 26, 23 April 2021 (2021-04-23), pages 265204, XP020367084, ISSN: 0022-3727, [retrieved on 20210423], DOI: 10.1088/1361-6463/ABF400

## Beschreibung

Die Erfindung betrifft ein integriertes Therapie- und Diagnostik-System (Theragnostik-System) zur Behandlung (Therapie) und Gewebebestimmung (Diagnostik). Insbesondere betrifft die Erfindung ein Theragnostik-System zur Erkennung von Gewebemerkmalen, insbesondere Gutartigkeit oder Bösartigkeit desselben während eines chirurgischen Eingriffs. Weiter betrifft die Erfindung ein Verfahren zur Auswertung von Daten vor, während und/oder nach der Operation zur Gewinnung von Information über das behandelte Gewebe.

Aus der EP 2 659 846 B1 ist eine Einrichtung bekannt, zu der ein chirurgisches Instrument mit einer Elektrode gehört, die an einen Generator angeschlossen ist, der die Elektrode mit hochfrequenter Wechselspannung versorgt. Zwischen der Elektrode und biologischem Gewebe wird ein Funken unterhalten, von dem Licht ausgeht. Dieses Licht wird mit einer Lichtaufnahmeeinrichtung aufgenommen und einer Analyseeinrichtung zugeführt, die eine Spektraluntersuchung vornimmt. Aus dem erzeugten Spektrum kann durch gezielte Analyse auch gegebenenfalls mit Mustererkennung und durch Vergleich mit in einer Datenbank abgelegten Lichtmerkmalen darauf geschlossen werden, ob das Gewebe gutartiges, benignes Gewebe oder entartetes, malignes Gewebe ist. Dies kann dem Chirurgen angezeigt werden.

Weiter ist aus der US 2007/0213704 A1 ein medizinisches Instrument zur Kaltabtragung von Gewebe durch sehr kurze Hochfrequenzfunken bekannt. Das von den Funken ausgehende Licht wird einem Spektralanalysator zugeführt, um anhand der erfassten Spektren Steuersignale zu erzeugen und eine Gewebeunterscheidung zu ermöglichen.

Die WO 2011/055369 A2 offenbart ein Katheter zur Plaqueabtragung aus Blutgefäßen. Der zur Abtragung der Plaques genutzte Funke erzeugt Licht, das über eine Lichtleitfaser einem Sensor zugeführt wird. Dieser erfasst die Phosphorlinie, um Plaques von lebenden Zellen zu unterscheiden.

Aus der EP 3 319 313 A1 geht ein Netzwerk hervor, das dazu eingerichtet ist, Daten von mehreren Punkten zusammenzuführen. Zu den Daten gehören Kamerabilder und andere Sensordaten, sowie Daten über die Umstände einer medizinischen Behandlung.

Weiterer Stand der Technik geht aus der DE 103 92 791 T5, der DE 198 60 689 C2, der US 2009/0326383 A1, der DE 42 31 677 A1, der WO 02/19243 A2, der US 6 026 323, der WO 03/020119 A2, der EP 0 650 694 A1, der DE 10 2020 105 853 A1, der US 2019/0223728 A1, der EP 2 491 882 A2 und der BASTIAN HILLEBRAND ET AL: "Tissue differentiation using optical emission spectroscopy for gastric mucosal devitalisation",JOURNAL OF PHYSICS D: APPLIED PHYSICS, INSTITUTE OF PHYSICS PUBLISHING, BRISTOL, GB, Bd. 54, Nr. 26, (2021-04-23) hervor.

Bei der praktischen Umsetzung der Gewebeerkennung durch Auswertung des von einem Funken ausgehenden Lichts treten Unsicherheiten auf, die eine sichere Gewebeerkennung erschweren.

Es ist Aufgabe der Erfindung, die automatische Gewebeerkennung bei und während der chirurgischen Einwirkung auf biologisches Gewebe zu verbessern.

Diese Aufgabe wird mit der Einrichtung nach Anspruch 1 gelöst:

Die erfindungsgemäße Einrichtung umfasst eine Chirurgiestation sowie eine Speicher- und Verarbeitungseinrichtung zur Verarbeitung von in der Chirurgiestation gewonnenen Daten. Die Speicher- und Verarbeitungseinrichtung kann Teil der Chirurgiestation oder eine gesonderte, von der Chirurgiestation entfernt aufgestellte Einrichtung sein, die mit der Chirurgiestation über ein Übertragungsnetzwerk in Verbindung steht. Die Einrichtung kann insbesondere auch mehrere Chirurgiestationen umfassen, die mittels einer Datenübertragungseinrichtung, beispielsweise eines Netzwerks, mit der gemeinsamen Speicher- und Verarbeitungseinrichtung verbunden oder verbindbar sind.

Zu der Chirurgiestation gehört mindestens ein elektrochirurgisches Instrument, das geeignet ist, chirurgisch auf biologisches Gewebe einzuwirken. Dazu weist das Instrument mindestens eine Elektrode auf. Weiter gehört zu der Chirurgiestation wenigstens ein Generator zur Versorgung des Instruments, insbesondere seiner Elektrode, mit einem zur Bewirkung des chirurgischen Effekts geeigneten elektrischen Strom, typischerweise einem hochfrequenten Strom mit einer Frequenz oberhalb 100 kHz, bevorzugt 300 kHz oder mehr. Die von dem Generator bereitgestellte und an die Elektrode des Instruments geleitete Spannung liegt typischerweise in einem Bereich von oberhalb 100 Volt bis zu mehreren 1000 Volt und ist wie der Strom entsprechend hochfrequent. Die hochfrequente Spannung kann wählbaren Modulationen unterliegen, um verschiedene chirurgische Effekte zu erzielen. Außerdem können verschiedene sonstige Parameter, wie Leistung, maximale Stromstärke, Modulation und dergleichen, angepasst werden. Solche Voreinstellungen des Generators zur Bewirkung unterschiedlicher chirurgischer Effekte werden als "Modes" bezeichnet.

Zu der Chirurgiestation gehört außerdem eine Erfassungseinrichtung, die dazu eingerichtet ist, das von dem zwischen der Elektrode und dem Gewebe erzeugten Funken ausgehende Licht aufzunehmen. Dieses Licht wird einer gleichfalls zur Chirurgiestation gehörenden Lichtanalyseeinrichtung zugeführt, die dazu eingerichtet ist, wenigstens ein optisches Merkmal des aufgenommenen Lichts zu erfassen. Das optische Merkmal kann insbesondere ein spektrales Merkmal sein, beispielsweise ein charakteristisches Spektrum, ein charakteristisches Teilspektrum, einzelne Wellenlängen des charakteristischen Spektrums oder eine oder mehrere aus dem Spektrum abgeleitete Größen. Weiter weist die Chirurgiestation eine Erfassungseinrichtung zur Gewinnung eines elektrischen Merkmals auf. Bei dem elektrischen Merkmal kann es sich um die Größe des Stroms, die Fluktuation des Stroms, die an der Elektrode anliegende Spannung, die Fluktuation der Spannung, die Modulation des Stroms und/oder der Spannung, den sich ergebenden Crestfaktor, die Impedanz des Gewebes, die Impedanz der aus Gewebewiderstand und Funkenwiderstand bestehenden Reihenschaltung, den Leistungseintrag, den Energieeintrag, den Phasenwinkel zwischen Strom und Spannung oder eine aus einer oder mehreren dieser Größen abgeleitete Rechengröße handeln.

Sowohl die optischen Merkmale als auch die elektrischen Merkmale werden der Speicher- und Verarbeitungseinrichtung zugeführt. Aus den optischen und bedarfsweise zusätzlich oder alternativ aus den elektrischen Merkmalen ermittelt die Speicher- und Verarbeitungseinrichtung durch Vergleich mit vorhandenen Daten ein Gewebelabel und übermittelt dieses an die Ausgabeeinrichtung der Chirurgiestation, um es dort zur Anzeige zu bringen. Zur Ermittlung des Gewebelabels, d.h. zur Gewebeidentifikation, nutzt das erfindungsgemäße System primär die optischen Merkmale. Ist auf deren Basis allein eine sichere Gewebeidentifikation nicht möglich, nutzt das System zusätzlich oder alternativ (sekundär) die elektrischen Merkmale. Dadurch wird die Gewebeidentifikation wesentlich sicherer und breiter einsetzbar als bei Nutzung ausschließlich optischer oder ausschließlich elektrischer Merkmale.

Bei dem Gewebelabel handelt es sich um ein Signal, das anzeigt, ob es sich bei dem behandelten, mit dem Funken in Kontakt stehenden Gewebe um malignes oder benignes Gewebe handelt. Die Ausgabeeinrichtung kann eine optische, akustische oder taktile Ausgabeeinrichtung sein, um dem Chirurgen oder sonstigem Behandler zu signalisieren, ob die Elektrode mit gesundem oder mit malignem Gewebe wechselwirkt. Im Fall einer Tumorresektion gewinnt er somit Information darüber, ob er im Tumor oder außerhalb eines Tumors schneidet. Indem die Speicher- und Verarbeitungseinrichtung nicht nur optische Merkmale, sondern auch elektrische Merkmale, und zwar insbesondere elektrische Merkmale, die eng mit dem Funken verknüpft sind, zur Gewebecharakterisierung heranzieht, kann die Gewebeunterscheidung trennschärfer und treffsicherer werden als aus dem Stand der Technik bekannt.

Die Lichtanalyseeinrichtung umfasst eine Spektralanalyseeinrichtung, die dazu eingerichtet ist als optisches Merkmal das Spektrum, Teile des Spektrums oder eine aus dem Spektrum oder aus Teilen des Spektrums abgeleitete Größe zu ermitteln. Dazu kann die Chirurgiestation eine lokale Verarbeitungseinrichtung aufweisen. Steht die Speicher- und Verarbeitungseinrichtung, in der Referenzspektren abgespeichert sind, außerhalb der Chirurgiestation, erfolgt somit die Bestimmung des optischen Merkmals lokal und es wird lediglich das optische Merkmal, nicht aber das gesamte Spektrum zu der entfernt stehenden Speicher- und Verarbeitungseirichtung übertragen. Dies reduziert den Datenübertragungsaufwand und erhöht somit die Reaktionsgeschwindigkeit des gesamten Systems.

Die Spektralanalyseeinrichtung weist außerdem einen Güteestimator auf, der die Verlässlichkeit des gewonnen optischen Merkmals bewertet und dem optischen Merkmal die gewonnene Bewertung zuordnet. Der Güteestimator nutzt zur Bewertung des gewonnenen optischen Merkmals ein Gütekriterium. Das Gütekriterium kann von der Art und Weise abhängen, wie das optische Merkmal gewonnen wird. Beispielsweise kann das optische Merkmal ein oder mehrere Spektrallinien oder Spektralbereiche sowie insbesondere deren Maximalintensitäten, das heißt Peaks bewerten, die sich aus dem Grundrauschen des Spektrums abzeichnen. Die typischen Peaks können beispielsweise die Kalziumlinie, die Magnesiumlinie, die Zinklinie oder auch Banden sein, die von Molekülfragmenten herrühren, zum Beispiel CN, N₂⁺, CH, CC, NH etc. Zeichnen sich solche Spektrallinien oder Peaks über dem Grundrauschen nur schwach ab, hat das gewonnene optische Merkmal, das auf diesen Peaks beruht, eine geringe Güte. Zeichnen sich gesuchte Linien jedoch deutlich ab, hat das optische Merkmal eine hohe Güte. Das von dem Güteestimator gelieferte Gütekriterium kann ein Digitalsignal (gültig/ungültig) oder auch ein analoges Signal sein, beispielsweise ein zwischen 0 und 1 liegender Wert, der zur Bewertung der Aussagekraft des optischen Merkmals herangezogen werden kann.

In einer bevorzugten Ausführungsform ist die Speicher- und Verarbeitungseinrichtung dazu ausgebildet, das Gewebelabel mit verminderter Rücksicht oder ohne Rücksicht auf das optische Merkmal anhand des elektrischen Merkmals festzulegen, wenn das Gütekriterium eine geringe oder fehlende Verlässlichkeit des optischen Merkmals anzeigt. Damit ist selbst mit minderwertigen (d.h. wenig charakteristischen) Spektren noch eine Gewebeunterscheidung möglich. Die Speicher- und Verarbeitungseinrichtung kann dabei weiter dazu ausgebildet sein, ein Warnsignal bereitzustellen, das an die Chirurgiestation übermittelt und dort in der Ausgabeeinrichtung ausgegeben wird, um dem Behandler darauf hinzuweisen, dass die Verlässlichkeit des ausgegebenen Gewebelabels gemindert ist.

Das erfindungsgemäße System kann als selbstlernendes System ausgebildet sein, das in der Lage ist, verschiedene Daten zu einer Datensammlung zusammenzuführen. Beispielsweise kann in der Chirurgiestation eine Eingabeeinrichtung eingerichtet sein, über die die Speicher- und Verarbeitungseinrichtung die Art des zu behandelnden Gewebes (zum Beispiel Muskelgewebe, Fettgewebe, Lungengewebe, Organgewebe, wie zum Beispiel Lebergewebe, Nierengewebe usw.) eingebbar sind. Die Eingabeeinrichtung kann weiter dazu vorgesehen und eingerichtet sein, weitere Daten, z.B. Metadaten, wie Patientendaten, aufzunehmen und an die Speicher- und Verarbeitungseinrichtung weiterzuleiten. Die Eingabeeinrichtung ist zur Eingabe von bestimmten Daten eingerichtet, indem sie für die Daten spezifische Eingabemasken anzeigt, in die die betreffenden Daten eintragbar oder importierbar sind.

Die Speicher- und Verarbeitungseinrichtung kann weiter dazu eingerichtet sein, die während der Operation gewonnenen optischen und elektrischen Merkmale aufzuzeichnen. Außerdem kann die Eingabeeinrichtung dazu eingerichtet sein, dass ein histologischer Befund eingegeben werden kann, der gleichfalls an die Speicher- und Verarbeitungseinrichtung übermittelt und den optischen und elektrischen Merkmalen zugeordnet wird. Der entstehende Rohdatensatz kann von der Speicher- und Verarbeitungseinrichtung dann bei weiteren Einsätzen verifiziert und schließlich als Referenzdatensatz zur Gewebeidentifikation folgender Operationen genutzt werden. Die Datensätze können spezifisch für individuelle Patienten oder auch für bestimmte Patiententypen oder Klassen bereitgehalten werden. Patiententypen oder Klassen können nach Geschlecht, Alter, Gewicht, Allgemeinzustand, Vorerkrankungen, Suchtmittelabhängigkeiten oder ähnlichen Kriterien festgelegt sein.

Eine entsprechende Eingabeeinrichtung kann auch außerhalb der Chirurgiestation vorgesehen sein, um zur Füllung der Datenbank Labordaten zu nutzen. Beispielsweise können explantierte Gewebe histologisch untersucht und im Labor probeweise mit einem elektrischen Instrument behandelt werden, um die dabei entstehenden elektrischen und optischen Merkmale zu ermitteln und in der Datenbank abzulegen.

Weiter ist es möglich, den Betrieb des Generators hinsichtlich wenigstens eines Betriebsparameters anhand des optischen Merkmals zu steuern. Beispielsweise kann somit die Spannung, der Strom, die Modulationsart, die Modulationstiefe, bei Puls-Pause-Modulation das Puls-Pause-Verhältnis, der Crest-Faktor, der Leistungseintrag und/oder der Energieeintrag in das biologische Gewebe gesteuert werden. Dabei ist es insbesondere möglich, den Generator so zu steuern, dass die optischen Merkmale möglichst eine hohe Güte aufweisen, so dass eine gute Verlässlichkeit des aufgrund der optischen und elektrischen Merkmale gewonnen Befunds gegeben ist.

Auch ist es möglich, den Generator anhand der Wünsche des Chirurgen zur Bewirkung des gewünschten chirurgischen Effekts zu steuern, wobei er dazu zum Beispiel eine erste Betriebsart einnimmt. Es kann sein, dass in dieser Betriebsart das optische Merkmal nur mit geringer Güte gewonnen werden kann. Dabei kann weiter vorgesehen sein, dass das System dazu eingerichtet ist, den Generator kurzzeitig in einer zweiten Betriebsart zu betreiben, die von dem Chirurgen nicht gewünscht und eingestellt ist, bei der jedoch das optische Merkmal eine hohe Güte aufweist. Die Umschaltung in diese Betriebsart erfolgt dabei vorzugsweise so kurzzeitig, dass der Chirurg subjektiv an dem von ihm geführten Instrument kein geändertes Verhalten oder jedenfalls kein signifikant geändertes Verhalten hinsichtlich des von ihm gewünschten chirurgischen Effektes am Gewebe feststellt. In der zweiten Betriebsart können elektrische Parameter, wie zum Beispiel die Spannung, der Strom, die Leistung, der Crest-Faktor, die Kurvenform oder ähnliches kurzzeitig, das heißt für weniger als 100 Millisekunden, vorzugsweise weniger als 10 Millisekunden und noch bevorzugter weniger als 1 Millisekunde verändert werden. Die Veränderung ist vorzugsweise eine Veränderung, die zu einer verbesserten Aussagekraft des erzeugten Spektrums führt. Der Generator kann darauf eingerichtet sein, diese Veränderung periodisch oder von Zeit zu Zeit anlassbezogen durchzuführen.

Das erfindungsgemäße System gestattet die Verknüpfung einer Chirurgiestation mit einer Theragnostik-Cloud. In der Chirurgiestation werden mittels einer Lichtanalyseeinrichtung optische Merkmale gewonnen und der Cloud zugeführt. In dieser findet eine integrierte selbstlernende Datenanalyse statt. Damit kann die Chirurgiestation und die Tätigkeit des Chirurgen überwacht und dynamisch bestenfalls patientenspezifisch adaptiert werden. Auch kann ein OP-Roboter ganz oder teilweise automatisch gesteuert werden. Es können sowohl zentral, als auch dezentral gelagerte Eingabe- und Annotierungssysteme als Datenquelle genutzt werden. Das cloud-basierte Theragnostik-System umfasst einen Cloud-basierten Datenspeicher mit einer auf maschinellem Lernen basierenden Datenverarbeitungseinheit. Der Datenspeicher erhält regelmäßig optische Emissionsspektren, Bioimpedanzdaten und/oder weitere elektrische Daten von klinisch relevanten Geweben mit dazugehöriger histopathologischer oder anderer zusätzlicher Identifizierung aus lokalen Datenbanken oder direkt aus der Chirurgiestation. Weiter empfängt die OES-Cloud (optische Emissionsspektroskopie-Cloud) individuelle Daten, das heißt elektrische Daten, Bioimpedanzen, optische Emissionsspektren von Operationen aus Operationssälen. Das System ermittelt bei Operationen mit HF-Chirurgie kontinuierlich Daten von Peripheriegeräten, wie beispielsweise OP-Kameras, Operationsraumrobotik sowie Sensor- und Bildgebungssystemen und/oder diagnostischer Bildgebung, wie Ultraschall, optische Kohärenztomographie, diffuse elektrische Tomografie, Impedanztomographie, Elastographie oder dergleichen entweder direkt oder über ein Operationssaal-Managementsystem. Weiter kann das System Häufungen von ungültigen Systemdaten, insbesondere HFchirurgischen Systemdaten identifizieren, die zur störungsfreien Theragnostik erforderlich sind. Sollten gehäuft oder länger ungültige Systemdaten ermittelt werden, kann ein entsprechendes Alarmsignal gegeben werden. Durch kontinuierliche Dateneingabe und Klassifizierung derselben für die Diagnostik anhand der elektrischen und optischen Merkmale wird die Gewebeerkennung kontinuierlich verbessert. Dies kann durch maschinelle Lernalgorithmen erfolgen. Aus den gewonnenen elektrischen und optischen Merkmalen können Steuerungsparameter für die robotergestützte Chirurgie gewonnen und live an Endgeräte übertragen werden. Außerdem kann die Speicher- und Verarbeitungseinrichtung eine Bibliothek von elektrischen und optischen Merkmalen in Verbindung mit Gewebemerkmalen und Patientenmerkmalen unterhalten und optimale Einstellungen des Generators (HF-Einstellungen) für laufende Eingriffe vorschlagen. Somit ist eine personalisierte Medizin durchführbar.

Einzelheiten des erfindungsgemäßen Theragnostik-Systems ergeben sich aus der Beschreibung nachfolgender Ausführungsbeispiele unter Zuhilfenahme der Zeichnung mit folgenden Figuren:
Figur 1 ein einfaches lokales Theragnostik-System in schematischer Übersichtsdarstellung,
Figur 2 ein cloud-basiertes Theragnostik-System in Übersichtsdarstellung,
Figur 3 ein Spektrum zur Gewinnung optischer Merkmale,
Figur 4 eine Veranschaulichung einer Datensammlung zur Bestimmung von Gewebemerkmalen,
Figur 5 ein Theragnostik-System mit einem verbesserten Generator,
Figur 6 Generatorausgangsimpulse des Generators nach Figur 5.

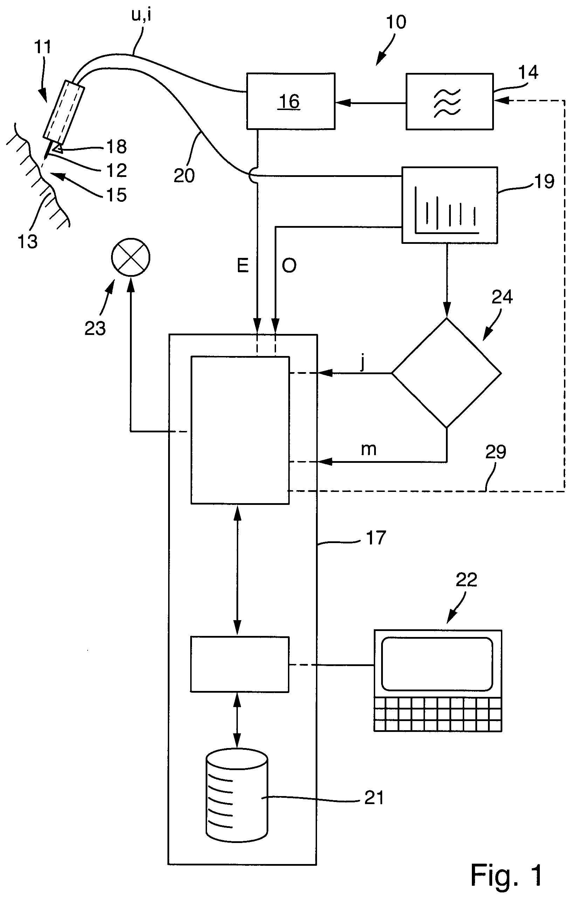

Figur 1 zeigt eine Chirurgiestation 10 insbesondere im Hinblick auf Datenerfassung und Verarbeitung in sehr schematisierter Darstellung. Die Chirurgiestation 10 umfasst die nicht weiter veranschaulichten Bestandteile eines Operationssaals. Insbesondere umfasst sie ein chirurgisches Instrument 11 mit einer Elektrode 12 zur Einwirkung auf biologisches Gewebe 13 während eines chirurgischen Eingriffs. Die Elektrode 12 des Instruments 11 wird von einem Generator 14 mit Behandlungsspannung u und Behandlungsstrom i versorgt, die dem Instrument 11 über eine Leitung zugeführt werden. Die Behandlungsspannung u und der Behandlungsstrom i sind vorzugsweise hochfrequent mit einer Frequenz oberhalb 100 kHz, beispielsweise 300 oder 400 kHz oder irgendeiner anderen geeigneten Frequenz. Die Behandlungsspannung u und der Behandlungsstrom i sind vorzugsweise so bereitgestellt, dass an der Elektrode 12 ein mit dem Gewebe 13 wechselwirkender Funke 15 entsteht, der an dem Gewebe 13 einen gewünschten chirurgischen Effekt, wie beispielsweise eine Koagulation, einen Schnitt oder einen anderen Effekt bewirkt.

Der Generator 14 liefert die Behandlungsspannung u und den Behandlungsstrom i über eine Erfassungseinrichtung 16, die wenigstens ein elektrisches Merkmal E bestimmt. Ein solches elektrisches Merkmal E kann der Behandlungsstrom, die Behandlungsspannung, bei variablen Behandlungsspannungsfrequenzen die Frequenz der Behandlungsspannung oder des Behandlungsstroms, der Modulationsgrad der Behandlungsspannung, die Modulationsart der Behandlungsspannung bei pulspausegetasteter Behandlungsspannung oder Behandlungsstrom das Puls/Pause-Verhältnis, die Gewebeimpedanz, die Nichtlinearität der Funkenimpedanz, ein Funkensensorwert, der sich beispielsweise aus dem gemessenen Gleichstromanteil der realen Wechselspannung zusammensetzt oder eine Kombination dieser Größen oder eine aus einer Kombination einer oder mehrerer dieser Größen abgeleiteten Größe sein. Das elektrische Merkmal E kann auch mehrere solcher Größen umfassen. Es wird an eine Speicher- und Verarbeitungseinrichtung 17 geliefert.

Die Chirurgiestation 10 weist außerdem eine Lichtaufnahmeeinrichtung 18 auf, die insbesondere dazu eingerichtet ist, das von dem Funken 15 ausgehende Licht aufzunehmen. Die Lichtaufnahmeeinrichtung 18 kann als Teil des Instruments 11 oder auch gesondert von diesem ausgebildet sein. Die Lichtaufnahmeeinrichtung 18 ist mit einer Lichtanalyseeinrichtung 19 verbunden, die Teil des Instruments 11 sein kann oder die alternativ, wie es Figur 1 schematisch andeutet, über einen Lichtleiter 20 mit der Lichtaufnahmeeinrichtung 18 verbunden sein kann. Die Lichtanalyseeinrichtung 19 ist dazu eingerichtet, aus dem aufgenommenen Funkenlicht ein oder mehrere optische Merkmale O zu ermitteln.

Das optische Merkmal kann durch Spektralanalyse und/oder Auswertung der Spektralanalyse des Funkenlichts gewonnen worden sein. Das optische Merkmal kann auch das Spektrum selbst sein, das heißt optische Signale oder Daten, die das Spektrum des Funkenlichts oder ein Teilspektrum desselben repräsentieren. Das optische Merkmal O kann zum Beispiel mehrere Einzelmerkmale O₁, O₂ ... Oₙ umfassen, die an die Speicher- und Verarbeitungseinrichtung 17 geliefert werden. Ebenso kann das elektrische Merkmal E eine Anzahl von elektrischen Einzelmerkmalen E₁, E₂ ... Eₙ umfassen. Die optischen Einzelmerkmale O₁, O₂, ... Oₙ können beispielsweise die Intensitäten bestimmter in dem Spektrum S des Funkenlichts vorkommende Intensitäten von einzelnen Wellenlängen λ₁, λ₂, ... λₙ sein. Die optischen Einzelmerkmale O₁, O₂, Oₙ können auf bestimmte Emissionslinien charakteristischer chemischer Elemente beruhen, wie beispielsweise Atomemissionslinien des Calciums, Magnesiums, Zinks oder auch Emissionslinien, Emissionsbanden oder Teilspektren von charakteristischen Molekülfragmenten, wie beispielsweise CN, N₂⁺, CH, CC, NH etc. sein.

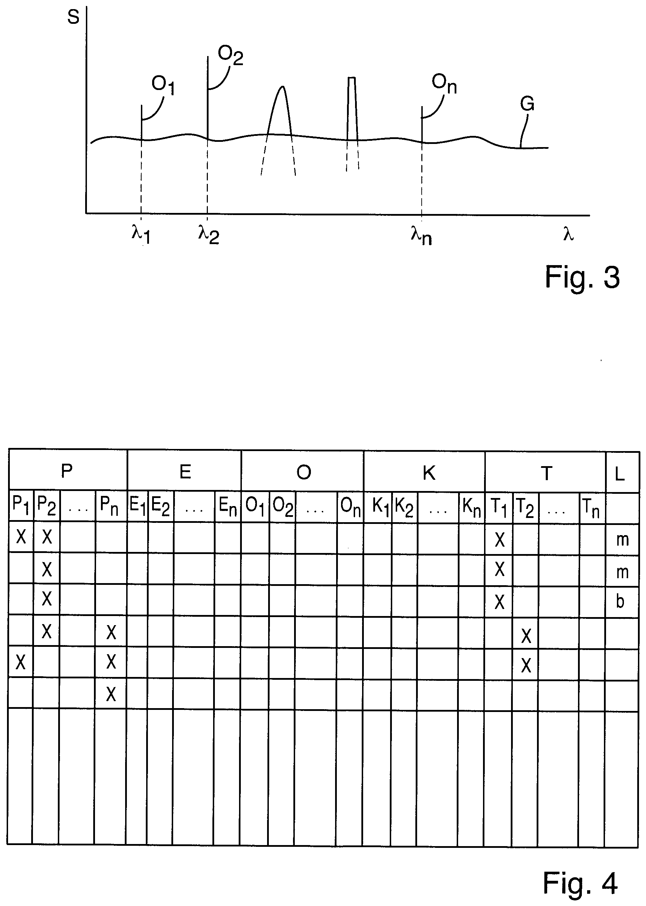

Die Speicher- und Verarbeitungseinrichtung 17 weist einen Massenspeicher 21 auf, der die von einer oder mehreren Chirurgiestationen 10 gelieferten Daten geordnet fasst. Die Daten können beispielsweise nach einer Tabelle organisiert sein, wie sie in Figur 4 angedeutet ist. In diese Tabelle finden die elektrischen Merkmale E ebenso Eingang, wie die optischen Merkmale O. Diese können verschiedenen Patienten P (P₁, P₂, P₃ ... Pₙ) und verschiedenen Gewebetypen T (T₁, T₂, T₃ ... Tₙ) zugeordnet sein. Die Patienten P₁ bis Pₙ können individuelle Patienten oder bestimmte Patientengruppen sein, die nach gemeinsamen Merkmalen geordnet sind, beispielsweise nach Alter, Gewicht, Körperfettanteil, Krankheitsstatus oder ähnlichem. Die Tabelle nach Figur 4 kann weitere Einträge enthalten, die dort nicht angegeben sind.

Zum Anlegen des Datensatzes gemäß der Tabelle nach Figur 4 in dem Massenspeicher 21 kann die Speicher- und Verarbeitungseinrichtung 17 mit einer Eingabeeinheit 22 verbunden sein, über die den Patienten oder Patientengruppen sowie den bei einer chirurgischen Einwirkung auf das Gewebe 13 auftretenden elektrischen Merkmalen E und optischen Merkmalen O, Krankheitsmerkmale K, (K₁, K₂ ... Kₙ) zugeordnet werden können. Außerdem kann jedem Datensatz ein Label L zugeordnet werden, das anzeigt, ob malignes Gewebe m oder benignes Gewebe b vorliegt. Als Datensatz wird in Figur 4 jeweils eine Zeile der dargestellten Tabelle verstanden. Die Tabelle kann jedoch wie schon erwähnt noch mehr Spalten und Zeilen aufweisen als dargestellt und deutlich mehr Daten umfassen, beispielsweise verwendete Instrumente und Geräte, Einstellung der Geräte, behandelnde Personen usw.

Die Speicher- und Verarbeitungseinrichtung 17 kann außerdem mit einer Anzeige- und/oder akustischen Einrichtung 23 verbunden sein, die dem Chirurgen einen Hinweis über das Label des Gewebes gibt, das der Funken 15 berührt. Außerdem kann die Speicher- und Verarbeitungseinrichtung 17 einen Estimator 24 aufweisen oder mit einem solchen verbunden sein, der seinerseits mit der Lichtanalyseeinrichtung 19 verbunden ist und von dieser entweder die optischen Merkmale O oder ein anderes Signal erhält, das jedenfalls die Aussagekraft der optischen Merkmale O kennzeichnet. Der Estimator kann beispielsweise den Signal-Rausch-Abstand oder das Signal/Rausch-Verhältnis zwischen einem aus Figur 3 ersichtlichen Grundrauschen G und den einzelnen optischen Merkmalen O₁, O₂ bis Oₙ kennzeichnen. Sind alle oder einzelne optische Merkmale O₁ bis Oₙ nicht ausreichend von dem Grundrauschen G unterschieden, sinkt die Verlässlichkeit der optischen Merkmale O deutlich ab und kann auf Null zurückgehen.

Der Estimator 24 gibt der Speicher- und Verarbeitungseinrichtung 17 ein entsprechendes Signal, im einfachsten in Figur 1 dargestellten Fall- ein Ja-Nein-Signal.

Anstatt des Signal/Rausch-Verhältnisses kann der Estimator auch ein Signal/Signal-Verhältnis bilden, in dem zwei oder mehr optische Merkmale des aufgenommenen Spektrums zueinander ins Verhältnis gesetzt werden. Sofern dieses Verhältnis einen zuvor definierten Grenzwert unter- oder überschreitet oder in einem zuvor festgelegten Intervall liegt, wird das Spektrum als gültig oder ungültig bewertet.

In einer einfachen Variante kann der Estimator 24 auch nur die Intensität eines oder mehrerer optischer Merkmale zur Bewertung der Güte heranziehen. Die Intensität kann dabei sowohl der Maximalwert des optischen Merkmals als auch das Integral des optischen Merkmals in einem definierten Wellenlängenbereich sein. Liegt der ermittelte Wert ober- oder unterhalb eines zuvor definierten Grenzwerts oder innerhalb eines zuvor definierten Bereichs, wird das Spektrum als gültig oder ungültig bewertet.

Der Estimator 24 kann auch darauf ausgelegt sein, das Vorhandensein bestimmter optischer Merkmale zu überprüfen, die auf eine Wechselwirkung mit nicht-biologischem Material schließen lassen. Insbesondere sind hier die optischen Merkmale von Metallen geeignet, um versehentliche Wechselwirkungen mit anderem Operationsgeräten, bspw. metallischen Klemmen, zu charakterisieren und derartige Spektren als ungültig zu bewerten.

Es können außerdem Estimatoren 24 anderer Bauart Anwendung finden. Z.B. kann der Estimator 24 darauf eingerichtet sein, das erfasste Spektrum mit vielen verschiedenen, zu einem Mustervorrat gehörigen Spektren abzugleichen. Dies kann durch Mustervergleich, Kreuzkorrelationsanalyse, Ähnlichkeitsanalyse oder anderweitige Verfahren erfolgen. Der Estimator 24 kann darauf eingerichtet sein, das aufgenommene Spektrum als nicht vertrauenswürdig zu kennzeichnen, wenn es mit keinem der vorhandenen Musterspektren korreliert ist oder zu keinem der vorhandenen Musterspektren ähnlich ist.

Es besteht auch die Möglichkeit, dass die hier jeweils separat aufgeführten Estimatoren beliebig miteinander kombiniert werden, um die Qualität der Bewertung zu erhöhen.

Die insoweit beschriebene Chirurgiestation 10 arbeitet beispielsweise wie folgt:

Der Speicher- und Verarbeitungseinrichtung 17 ist zunächst der spezielle Patient oder die Zugehörigkeit des Patienten zu einer Patientengruppe P₁ oder P₂ ... oder Pₙ mitgeteilt worden. Diese Mitteilung kann durch eine an dem Patienten angebrachte Kennung einen Barcode, eine Nummer, eine Patientenkarte oder auch durch manuelle Eingabe einer Patientenidentifikation, beispielsweise eines Namens über die Eingabeeinrichtung 22 erfolgen. Es wird beispielsweise davon ausgegangen, dass der Patient zur Patientengruppe P₂ gehört, die Teil der Veranschaulichung in Figur 4 ist. Dies bedeutet, dass für diesen Patienten nur diejenigen Datensätze in Frage kommen, die zu seiner Patientengruppe gehören. Beginnt nun die Operation, werden sowohl die elektrischen Merkmale E als auch die optischen Merkmale O bestimmt. Außerdem kann zumindest optional der Gewebetyp auf den eingewirkt wird, beispielsweise Lungengewebe T₁ eingegeben worden sein. Dies bedeutet, dass in dem Beispiel nach Figur 4 nur die ersten drei Datensätze (die ersten drei Zeilen) zur weiteren Analyse in Frage kommen. Es werden nun die elektrischen Merkmale E und die optischen Merkmale O erfasst und mit der Tabelle nach Figur 4 abgeglichen, wobei diese im praktischen wesentlich mehr Zeilen und Datensätze aufweist als dargestellt. Anhand der einzelnen elektrischen Merkmale E₁ bis Eₙ und optischen Merkmale O₁ bis Oₙ kann nun relativ sicher darauf geschlossen werden, ob das von dem Funken 15 berührte Gewebe mit einem malignen Label m oder einem benignen Label b zu versehen ist. Entsprechendes kann von der Anzeigeeinrichtung 23 dann zur Anzeige gebracht werden.

Zusätzlich können gerade bei großen Datenmengen und Datensätzen weitere Randbedingungen, wie beispielsweise Krankheiten K₁ bis Kₙ oder weitere Einflussfaktoren, Berücksichtigung und somit Eingang in die Datensätze nach Figur 4 finden.

Es ist möglich, dem System nach Figur 1 bei Operationen den histologischen Befund des Gewebes mitzuteilen und somit weitere Datensätze zu erstellen, die jeweils einen Zusammenhang zwischen den elektrischen Merkmalen E, den optischen Merkmalen O und dem zugehörigen Label L herstellen. Damit kann das System mit zunehmender Benutzungsdauer mehr und mehr Lernen und seine Prognosen verfeinern.

Außerdem kann das System aufgrund des Estimators 24 davon abgehalten werden, auf Basis unsicherer optischer Merkmale O Fehlvorschläge zu machen. Nimmt die Vertrauenswürdigkeit der optischen Merkmale O ab oder ist diese letztendlich nicht vorhanden, kann das System, das heißt die Speicher- und Verarbeitungseinrichtung 17, auch allein anhand der elektrischen Merkmale E im Zusammenhang mit den sonstigen vorhandenen Merkmalen mit einiger Sicherheit noch ein gültiges Label L (nämlich m oder b) ausgeben.

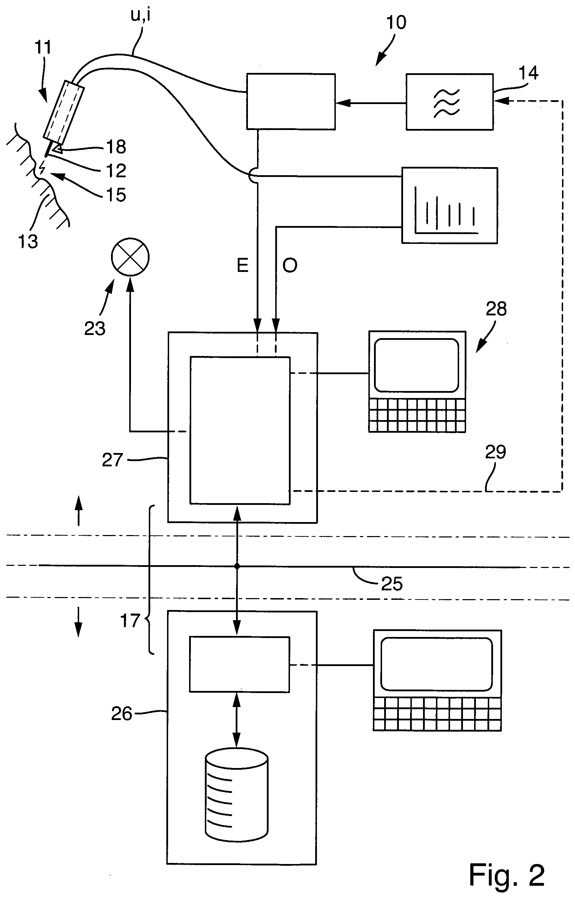

Das insoweit beschriebene System 10 kann gemäß Figur 2 auch mehrere Chirurgiestationen 10 umfassen, die über eine Datenfernverbindung 25 mit einem zentralisierten, als Cloud 26 bezeichneten Teil der Speicher- und Verarbeitungseinrichtung 17 verbunden sind. Der in jeder Chirurgiestation 10 verbleibende Teil der Speicher- und Verarbeitungseinrichtung 17 wird durch eine lokale Verarbeitungseinrichtung 27 gebildet. Im vorliegenden Ausführungsbeispiel weist die lokale Verarbeitungseinrichtung 27 auch eine Eingabeeinrichtung 28 auf, über die in der Chirurgiestation 10 Eingaben, zum Beispiel über den Patienten, seinen Krankheitsstatus, histologische Befunde oder dergleichen gemacht werden können. Diese Angaben übermittelt die Verarbeitungseinrichtung 27 ganz oder teilweise an die Cloud 26. Ebenso übermittelt die Verarbeitungseinrichtung 27 die elektrischen und optischen Merkmale E und O ganz oder teilweise an die Cloud 26. In der Cloud 26 werden die erhaltenen Patientenmerkmale, sowie die elektrischen und die optischen Merkmale E, O anhand der Datensammlung gemäß Figur 4 untersucht und es wird ein entsprechendes Label zur Kennzeichnung der Gutartigkeit oder der Bösartigkeit des behandelnden Gewebes an die Verarbeitungseinrichtung 27 zurückgeliefert. Das Ergebnis kann mit der Anzeigeeinrichtung 23 optisch oder akustisch oder taktil zur Kenntnis des Operateurs gebracht werden.

Die Chirurgiestation alleine nach Figur 1 oder die Chirurgiestationen 10 nach Figur 2 in Verbindung mit der Cloud 26 bilden ein Theragnostik-System das sowohl die Therapie, als auch die Diagnostik unterstützt.

Die Chirurgiestation 10 nach Figur 1 oder Figur 2 kann außerdem eine Rückkopplung zwischen der Speicher- und Verarbeitungseinrichtung 17 und dem Generator 14 aufweisen. Beispielsweise können elektrische Parameter des Generators 14, wie beispielsweise die von dem Instrument 11 auf das Gewebe 13 übertragene Leistung, die Spannung, der Strom, die Modulationsform, die Wellenform der Spannung oder des Stroms oder andere elektrische Parameter in Abhängigkeit von den erfassten elektrischen und/oder optischen Merkmalen E und/oder O variiert werden. Dies beispielsweise um Fehlbehandlungen zu verhindern oder diagnostische Möglichkeiten zu verbessern. Beispielsweise kann das System 10 dazu eingerichtet sein, bei Erfassung einer Berührung malignen Gewebes den Generator 14 abzuschalten oder auf eine andere zum Beispiel auch höhere Leistungsstufe zu fahren. Auch kann das System 10 dazu eingerichtet sein, die elektrischen Parameter dauerhaft oder vorübergehend oder auch nur ganz kurzzeitig und sporadisch oder auch wiederkehrend so zu verändern, dass die Aussagekraft der optischen Merkmale O steigt. Zum Beispiel kann in einem ersten Mode M1, bei dem die Aussagekraft der optischen Merkmale O gering ist, ganz kurzzeitig, beispielsweise wenige Millisekunden auf einen anderen Mode M2 umgeschaltet werden, der eine höhere Aussagekraft der optischen Merkmale O liefert. Die Umschaltung kann durch eine kurzzeitige Spannungsänderung oder auch Modulationsänderung bewirkt werden. Die dazu vorgesehene Rückkopplung 29 ist insbesondere dann wirksam, wenn in dem Datensatz nach Figur 4 zugehörig zu den elektrischen Merkmalen E und optischen Merkmalen O auch eine Kennzeichnung K abgespeichert wird, die die Verlässlichkeit der optischen Merkmale O kennzeichnet.

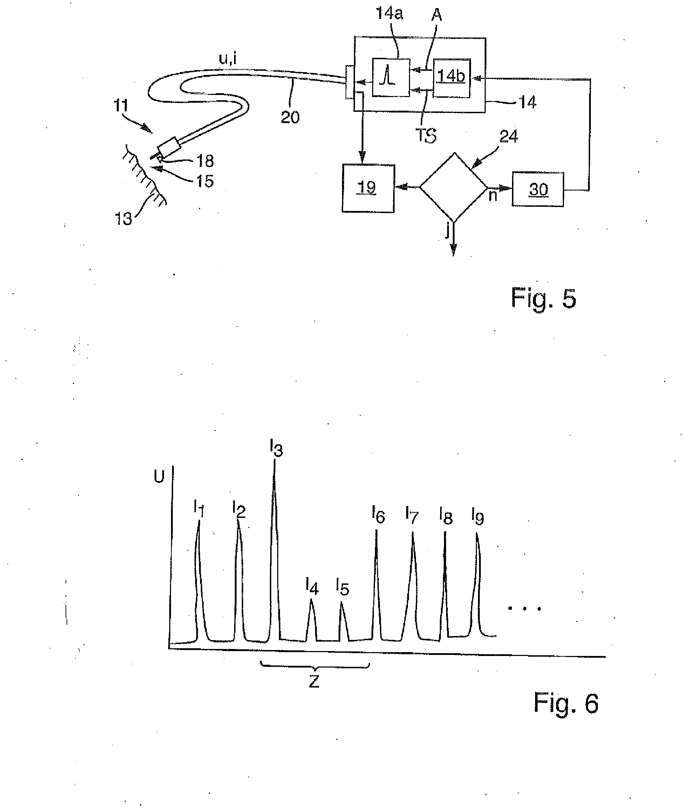

Figur 5 veranschaulicht wesentliche Teile eines Generators 14 einer Chirurgiestation, der dazu eingerichtet ist, die Qualität der optischen, gewebekennzeichnenden Signale zu verbessern. Dieser Generator 14 ist wiederum zum Betrieb des Instruments 11 eingerichtet, das mittels des auf das Gewebe 13 einwirkenden Funkens 15 einerseits eine chirurgische Wirkung erzielt und andererseits Licht erzeugt, das über die Lichtaufnahmeeinrichtung 18 und die Leitung 20 der Lichtanalyseeinrichtung 19 zugeführt wird.

Die Besonderheit des Generators 14 nach Figur 5 liegt darin, dass dieser dazu eingerichtet ist, Einzelimpulse I₁, I₂, I₃, I₄, I₅, I₆ ... abzugeben, die zusammen eine monopolare oder bipolare HF-Schwingung bilden. Dazu ist der Generator 14 in einen Impulsgenerator 14a und einen Taktgeber 14b unterteilt, der dem Impulsgenerator 14a einerseits ein Taktsignal TS zuleitet, das jeden Einzelimpuls I₁ bis I₆ ... auslöst. Andererseits ist der Taktgeber 14b dazu eingerichtet, dem Impulsgenerator 14a ein Amplitudensignal A zuzuleiten, dass die Größe des jeweilig erzeugten Einzelimpulses I₁ bis I₆ ... festlegt. Der Impulsgenerator kann einen oder mehrere Sperrwandler umfassen, die jeweils gleichzeitig oder zu unterschiedlichen Zeitpunkten zur Abgabe eines Ausgangsimpulses veranlassbar sind und bei Empfang eines Taktsignals einen einzelnen Ausgangsimpuls abgeben. Durch gleichzeitige Abgabe mehrerer Ausgangsimpulse durch mehrere Sperrwandler werden größere Ausgangsimpulse erzeugt, weil sich die Ausgangsimpulse der einzelnen Sperrwandler addieren.

Der Taktgeber 14b ist dabei dazu eingerichtet, das Taktsignal TS und das Amplitudensignal A so vorzugeben, dass der gewünschte chirurgische Effekt erzeugt wird. Soll beispielsweise eine HF-Impulsfolge mit konstanter Amplitude A erzeugt werden, wird bei konstantem Amplitudensignal A eine Folge von Taktsignalen TS an den Impulsgenerator 14a geliefert. Fordert der gewünschte chirurgische Mode hingegen eine unterbrochene Impulsfolge mit Impulsen konstanter Amplitude, wird bei konstantem Amplitudensignal A eine entsprechende unterbrochene Folge von Taktsignalen TS an den Impulsgenerator 14 geliefert.

Die Besonderheit des in Figur 5 veranschaulichten Generators 14 liegt in seiner Verbindung zu der Lichtanalyseeinrichtung 19 und dem Estimator 24. Beide können nach jedem der vorstehend beschriebenen Prinzipien arbeiten. Der Estimator 24 ist jedoch zusätzlich mit einem Modekatalogspeicher 30 verbunden, in dem Modedatensätze gespeichert sind, die verschiedene Impulsfolgen I₁ ... I₆ ... charakterisieren, die zu verschiedenen Behandlungsmodalitäten gehören. Insbesondere enthält der Modekatalogspeicher 30 zu jedem gespeicherten Modedatensatz eine Kennung, die anzeigt, inwieweit die zugehörige Impulsfolge geeignet ist, ein auswertbares Spektrum zu liefern. Insbesondere sind in dem Modekatalogspeicher Daten für solche Impulsfolgen I₃ bis I₅ abgespeichert, die aussagekräftige Spektren liefern. Beispielsweise kann es sein, dass der Chirurg eine Behandlungsmodalität wünscht, die zu wenig aussagekräftigen Spektren führt. Im Beispiel ist dies durch eine Folge konstanter Impulse I₁, I₂ usw. veranschaulicht. Wenn nun die Erzeugung einer solchen Impulsfolge und der daraus resultierenden Funken 15 zu einer Lichterscheinung führen, deren von der Lichtanalyseeinrichtung 19 ermitteltes Spektrum von dem Estimator 24 als nicht aussagekräftig eingestuft wird, kann mit dieser Impulsfolge keine sichere optische Gewebeanalyse durchgeführt werden. Bei der Ausführungsform nach Figur 5 wird dadurch jedoch der Modekatalogspeicher 30 aktiviert, der ein entsprechendes Signal an den Taktgeber 14b sendet. Dieses Signal umfasst entweder Information über einen Einzelimpuls oder eine kurze Impulsfolge I₃, I₄, I₅, die zu einer Lichterscheinung mit besser auswertbarem Spektrum führen. Die resultierende Impulsfolge ist in Figur 6 veranschaulicht. Während der von dem Chirurg gewünschte Mode eine Folge konstant hoher Spannungsimpulse I₁, I₂, I₆ usw. mit einer Wiederholrate von zum Beispiel 5 µs erfordert, wird der Taktgeber 14b veranlasst, zu wiederkehrenden Zeitabständen, beispielsweise alle 0,5 Sekunden, eine Zwischenimpulsfolge Z in die Behandlungssignalfolge I₁, I₂, I₆ einzuschieben.

Die Zwischenimpulsfolge Z kann einen oder mehrere Impulse I₃, I₄, I₅ umfassen, die gleiche oder unterschiedliche Amplituden aufweisen und die in gleichen oder in unterschiedlichen Zeitabständen zu den Behandlungssignalimpulsen I₁, I₂, I₆ ... I₉ abgegeben werden. Die Anzahl, die Zeitabstände und die Größe der Zwischenimpulse I₃ bis I₅ sind dabei vorzugsweise so festgelegt, dass optimale aussagekräftige Spektren erhalten werden. Die zeitliche Länge der Zwischenimpulsfolge Z ist vorzugsweise so kurz festgelegt, dass der chirurgische, von den Behandlungsimpulsen I₁, I₂ sowie I₆ bis I₉ ... erhoffte Effekt wenig oder nicht verändert oder gar beeinträchtigt wird.

Ein erfindungsgemäßes Theragnostik-System umfasst eine Chirurgiestation 10 und eine Speicher- und Verarbeitungseinrichtung 17, die in einem geeigneten Speicher eine große Datenmenge enthält, in der Patientendaten und Behandlungsdaten, zum Beispiel in Gestalt elektrischer und optischer Merkmale E, O, zusammengeführt werden. Die elektrischen Merkmale E sind aus elektrischen Größen der Spannung und des Stroms abgeleitet, mit denen ein Instrument 11 gespeist wird. Die optischen Merkmale sind aus dem Licht des Funkens 15 abgeleitet, der bei der Einwirkung auf das Gewebe 13 entsteht. Durch die Zusammenführung elektrischer Merkmale E und optischer Merkmale O in einer Datensammlung, beispielsweise Datenbank, die noch weitere Merkmale, wie zum Beispiel Gewebemerkmale und optional Patientenmerkmale enthält, kann mit großer Sicherheit automatisch ermittelt werden, ob das Instrument auf gesundes oder krankes Gewebe einwirkt. Die Vorhersagegenauigkeit kann durch maschinelles Lernen vergrößert werden, indem den Datensätzen zusätzlich zu den elektrischen Merkmalen E und den optischen Merkmalen O histologische Daten hinzugefügt werden. Bei einer bevorzugten Ausführungsform werden diese Daten in einer Cloud 26 gesammelt, die mit vielen Chirurgiestationen 10 verbunden ist. In der Cloud 26 können sich somit Daten sammeln, die in verschiedenen Chirurgiestation 10 gewonnen worden sind.

### Bezugszeichen:

- 10: Chirurgiestation
- 11: Instrument
- 12: Elektrode
- 13: biologisches Gewebe
- 14: Generator
- 14a: Impulsgenerator
- I₁, I₆...I₉: Behandlungsimpulse
- I₃...I₅: Zwischenimpulse
- 14b: Taktgeber
- TS: Taktsignal
- A: Amplitudensignal
- u: Behandlungsspannung
- i: Behandlungsstrom
- 15: Funke
- E: elektrisches Merkmal
- 16: Erfassungseinrichtung für elektrische Merkmale E
- 17: Speicher- und Verarbeitungseinrichtung
- 18: Lichtaufnahmeeinrichtung
- 19: Lichtanalyseeinrichtung
- 20: Lichtleiter
- O: optisches Merkmal
- P: Patientenidentifikator
- T: Gewebeidentifikator
- 21: Massenspeicher
- 22: Eingabeeinheit
- 23: Anzeigeeinrichtung
- 24: Estimator
- 25: Datenfernverbindung
- 26: Cloud
- 27: Verarbeitungseinrichtung
- 28: Eingabeeinrichtung
- 29: Rückkopplung
- 30: Modekatalogspeicher
- Z: Zwischenimpulsfolge

## Patentansprüche

1. Theragnostik-System:
mit mindestens einer Chirurgiestation (10), die mindestens aufweist:
- eine Lichtaufnahmeeinrichtung (18) zur Erfassung von Licht, das von einem Funken (15) ausgeht, der zwischen einer Elektrode (12) eines elektrochirurgischen Instruments (11) und biologischem Gewebe (13) erzeugt wird,
- eine Lichtanalyseeinrichtung (19), die dazu eingerichtet ist, wenigstens ein optisches Merkmal (O) des aufgenommenen Lichts zu erfassen,
- einen Generator (14) zur Bereitstellung einer elektrischen Spannung (u) zur Versorgung des Instruments (11) und dabei zur Speisung der Elektrode (12) des Instruments (11) mit elektrischem Strom (i),
- eine Ausgabeeinrichtung (23), und
mit einer Speicher- und Verarbeitungseinrichtung (17), die dazu eingerichtet ist, anhand des wenigstens einen optischen Merkmals (O) ein Gewebelabel (L) zu ermitteln und dieses an die Ausgabeeinrichtung (23) zur Ausgabe zu übermitteln,
**dadurch gekennzeichnet,**
**dass** die Chirurgiestation (10) weiter eine Erfassungseinrichtung (16) zur Bestimmung wenigstens eines elektrischen Merkmals (E) aufweist,
**dass** die Lichtanalyseeinrichtung (19) eine Spektralanalyseeinrichtung umfasst und einen Güteestimator (24) aufweist, der dazu eingerichtet ist, anhand eines Gütekriteriums die Verlässlichkeit des gewonnen optischen Merkmals zu bewerten und dem optischen Merkmal die gewonnene Bewertung zuzuordnen,
**dass** die Speicher- und Verarbeitungseinrichtung (17), weiter dazu eingerichtet ist, falls das optische Merkmal (O) zur Gewebeidentifikation nicht ausreicht, zusätzlich oder ersatzweise anhand des wenigstens einen elektrischen Merkmals (E) ein Gewebelabel (L) zu ermitteln und dieses an die Ausgabeeinrichtung (23) zur Ausgabe zu übermitteln, und
**dass** die Speicher- und Verarbeitungseinrichtung (17) dazu eingerichtet ist, das Gewebelabel (L) mit verminderter oder ohne Rücksicht auf das optische Merkmal (O) anhand des elektrischen Merkmals (E) zu ermitteln, wenn das Spektrum eine nicht ausreichende Güte aufweist.

2. System nach Anspruch 1, **dadurch gekennzeichnet, dass** die Spektralanalyseeinrichtung dazu eingerichtet ist, das Spektrum, Teile des Spektrums oder aus dem Spektrum oder aus Teilen des Spektrums abgeleitete Größen als optisches Merkmal (O) zu ermitteln.

3. System nach Anspruch 2, **dadurch gekennzeichnet, dass** die Lichtanalyseeinrichtung (19) oder die Speicher- und Verarbeitungseinrichtung (17) den Güteestimator (24) für das Spektrum, Teile des Spektrums oder daraus abgeleitete Größen aufweist oder mit einem solchen verbunden ist.

4. System nach Anspruch 3, **dadurch gekennzeichnet, dass** der Güteestimator darauf eingerichtet ist, den Signal-Rausch-Abstand wenigstens einer Spektrallinie (O₁) oder eines Spektralbereichs (O₁, O₂) zu bestimmen.

5. System nach einem der vorstehenden Ansprüche, **dadurch gekennzeichnet, dass** es eine Eingabeeinrichtung (22, 28) aufweist, über die Gewebeidentifikatoren (T) eingebbar sind, wobei die Eingabeeinrichtung (22, 28) mit der Speicher- und Verarbeitungseinrichtung (17) verbunden ist, um den Gewebeidentifikator (T) an diese zu übermitteln.

6. System nach einem der vorstehenden Ansprüche, **dadurch gekennzeichnet, dass** die Speicher- und Verarbeitungseinrichtung (17) eine Datensammlung umfasst, in der jeweils einem Set von optischen und elektrischen Merkmalen (O, E) wenigstens ein Gewebelabel (L) zugeordnet ist.

7. System nach Anspruch 5 oder 6, **dadurch gekennzeichnet, dass** der Gewebeidentifikator (T) anhand der über die Eingabeeinrichtung (22, 28) eingegebenen Eingaben festgelegt ist.

8. System nach einem der vorstehenden Ansprüche, **dadurch gekennzeichnet, dass** die Speicher- und Verarbeitungseinrichtung (17) Teil der Chirurgiestation (10) ist.

9. System nach einem der vorstehenden Ansprüche, **dadurch gekennzeichnet, dass** der Generator (14) hinsichtlich wenigstens eines Betriebsparameters anhand des optischen Merkmals (O) gesteuert ist.

10. System nach einem der vorstehenden Ansprüche, **dadurch gekennzeichnet, dass** es dazu eingerichtet ist, dass der Generator (14), wenn er in einer ersten Betriebsart (M1) betrieben wird, bei denen das optische Merkmal (O) keine zuverlässige Gewebeerkennung zulässt, wenigstens kurzzeitig in einer zweiten Betriebsart (M2) betrieben wird, bei der anhand des optischen Merkmals eine Gewebeerkennung möglich ist.

11. System nach einem der vorstehenden Ansprüche, **dadurch gekennzeichnet, dass** es mehrere Chirurgiestationen (10) umfasst.

12. System nach Anspruch 11, **dadurch gekennzeichnet, dass** ein Teil (26) der Speicher- und Verarbeitungseinrichtung (17) über eine Datenübertragungseinrichtung (25) mit der Chirurgiestation (10) verbunden ist.

13. System nach einem der vorstehenden Ansprüche, **dadurch gekennzeichnet, dass** die Speicher- und Verarbeitungseinrichtung (17) mit einer außerhalb der Chirurgiestation (10) positionierten Eingabeeinrichtung (28) verbunden ist.

## Claims

1. A theragnostic system:
having at least one surgical station (10) that comprises at least:
- a light receiving device (18) for detecting light emanating from a spark (15) generated between an electrode (12) of an electrosurgical instrument (11) and biological tissue (13),
- a light analysis device (19) that is configured to detect at least one optical feature (O) of the received light,
- a generator (14) for providing an electrical voltage (u) for supplying the instrument (11) and thereby for supplying the electrode (12) of the instrument (11) with electrical current (i),
- an output device (23), and
having a storage and processing device (17) that is configured to determine a tissue label (L) based on the at least one optical feature (0) and to transmit this to the output device (23) for output,
**characterized in that**
the surgical station (10) further comprises a detection device (16) for determining at least one electrical feature (E),
the light analysis device (19) includes a spectral analysis device and has a quality estimator (24) that is configured to assess the reliability of the obtained optical feature based on a quality criterion and to assign the obtained assessment to the optical feature, the storage and processing device (17) is further configured, if the optical feature (O) is not sufficient for tissue identification, to additionally or
alternatively determine a tissue label (L) based on the at least one electrical feature (E) and to transmit this to the output device (23) for output, and
the storage and processing device (17) is configured to determine the tissue label (L) with reduced or without consideration of the optical feature (O) based on the electrical feature (E) if the spectrum does not have sufficient quality.

2. The system according to claim 1, **characterized in that** the spectral analysis device is configured to determine the spectrum, portions of the spectrum or parameters derived from the spectrum or from portions of the spectrum as optical feature (0).

3. The system according to claim 2, **characterized in that** the light analysis device (19) or the storage and processing device (17) comprises or is connected to the quality estimator (24) for the spectrum, portions of the spectrum or parameters derived therefrom.

4. The system according to claim 3, **characterized in that** the quality estimator is configured to determine the signal-to-noise ratio of at least one spectral line (O₁) or a spectral range (O₁, O₂).

5. The system according to anyone of the preceding claims, **characterized in that** it comprises an input device (22, 28) via which tissue identifiers (T) can be entered, wherein the input device (22, 28) is connected to the storage and processing device (17) in order to transmit the tissue identifier (T) to the latter.

6. The system according to anyone of the preceding claims, **characterized in that** the storage and processing device (17) comprises a data collection in which at least one tissue label (L) is assigned to each set of optical and electrical features (0, E).

7. The system according to claim 5 or 6, **characterized in that** the tissue identifier (T) is defined based on the inputs entered via the input device (22, 28).

8. The system according to anyone of the preceding claims, **characterized in that** the storage and processing device (17) is part of the surgical station (10).

9. The system according to anyone of the preceding claims, **characterized in that** the generator (14) is controlled with regard to at least one operating parameter based on the optical feature (0).

10. The system according to anyone of the preceding claims, **characterized in that** it is configured so that the generator (14), if operated in a first operating mode (M1) in which the optical feature (O) does not allow reliable tissue identification, is operated at least for a short period in a second operating mode (M2) in which tissue identification is possible based on the optical feature.

11. The system according to anyone of the preceding claims, **characterized in that** it comprises a plurality of surgical stations (10).

12. The system according to claim 11, **characterized in that** a part (26) of the storage and processing device (17) is connected to the surgical station (10) via a data transmission device (25).

13. The system according to anyone of the preceding claims, **characterized in that** the storage and processing device (17) is connected to an input device (28) positioned outside the surgical station (10).

## Revendications

1. Système de théranostique :
comprenant au moins un poste chirurgical (10) qui présente au moins :
- un dispositif de réception de lumière (18) destiné à détecter la lumière émanant d'une étincelle (15) qui est générée entre une électrode (12) d'un instrument électrochirurgical (11) et des tissus biologiques (13),
- un dispositif d'analyse de lumière (19) qui est conçu pour détecter au moins une caractéristique optique (O) de la lumière reçue,
- un générateur (14) destiné à fournir une tension électrique (u) pour l'alimentation de l'instrument (11), et ainsi pour l'alimentation de l'électrode (12) de l'instrument (11) avec du courant électrique (i),
- un dispositif de sortie (23), et
comprenant un dispositif de mémoire et de traitement (17) qui est conçu pour déterminer un marqueur de tissus (L) à l'aide de la caractéristique optique (O), au nombre d'au moins une, et le transmettre au dispositif de sortie (23) aux fins de le délivrer,
**caractérisé en ce que**
le poste chirurgical (10) présente en outre un dispositif de détection (16) destiné à déterminer au moins une caractéristique électrique (E),
**en ce que** le dispositif d'analyse de lumière (19) comprend un dispositif d'analyse spectrale et présente un estimateur de qualité (24) qui est conçu pour évaluer la fiabilité de la caractéristique optique obtenue, à l'aide d'un critère de qualité, et pour associer l'évaluation obtenue à la caractéristique optique,
**en ce que** le dispositif de mémoire et de traitement (17) est en outre conçu pour procéder, dans le cas où la caractéristique optique (O) ne suffit pas pour l'identification des tissus, en supplément ou en remplacement, à l'aide de la caractéristique électrique (E), au nombre d'au moins une, à la détermination d'un marqueur de tissus (L) et à la transmission de celui-ci au dispositif de sortie (23), aux fins de le délivrer, et
**en ce que** le dispositif de mémoire et de traitement (17) est conçu pour déterminer le marqueur de tissus (L) avec une prise en compte réduite ou sans prise en compte de la caractéristique optique (O), à l'aide de la caractéristique électrique (E), si le spectre présente une qualité qui n'est pas suffisante.

2. Système selon la revendication 1, **caractérisé en ce que** le dispositif d'analyse spectrale est conçu pour déterminer le spectre, des parties du spectre ou des grandeurs dérivées du spectre ou de parties du spectre, en tant que caractéristique optique (O).

3. Système selon la revendication 2, **caractérisé en ce que** le dispositif d'analyse de lumière (19) ou le dispositif de mémoire et de traitement (17) présente l'estimateur de qualité (24) pour le spectre, des parties du spectre ou des grandeurs qui en sont dérivées, ou est relié à un tel estimateur.

4. Système selon la revendication 3, **caractérisé en ce que** l'estimateur de qualité est conçu pour déterminer le rapport signal/bruit d'au moins une ligne spectrale (O₁) ou d'un domaine spectral (O₁, O₂).

5. Système selon une des revendications précédentes, **caractérisé en ce qu'**il comporte un dispositif d'entrée (22, 28) qui permet de saisir des identificateurs de tissus (T), le dispositif d'entrée (22, 28) étant relié au dispositif de mémoire et de traitement (17) pour lui transmettre l'identificateur de tissus (T).

6. Système selon une des revendications précédentes, **caractérisé en ce que** le dispositif de mémoire et de traitement (17) comprend un recueil de données dans lequel au moins un marqueur de tissus (L) est associé respectivement à un ensemble de caractéristiques optiques et électriques (O, E).

7. Système selon la revendication 5 ou 6, **caractérisé en ce que** l'identificateur de tissus (T) est défini à l'aide des entrées saisies par l'intermédiaire du dispositif d'entrée (22, 28).

8. Système selon une des revendications précédentes, **caractérisé en ce que** le dispositif de mémoire et de traitement (17) fait partie du poste chirurgical (10).

9. Système selon une des revendications précédentes, **caractérisé en ce que** le générateur (14) est commandé à l'aide de la caractéristique optique (O), en ce qui concerne au moins un paramètre de fonctionnement.

10. Système selon une des revendications précédentes, **caractérisé en ce qu'**il est agencé pour que le générateur (14), lorsqu'il est exploité dans un premier mode de fonctionnement (M1) dans lequel la caractéristique optique (O) ne permet pas une identification fiable des tissus, soit exploité au moins brièvement dans un deuxième mode de fonctionnement (M2) qui permet une identification des tissus à l'aide de la caractéristique optique.

11. Système selon une des revendications précédentes, **caractérisé en ce qu'**il comprend plusieurs postes chirurgicaux (10).

12. Système selon la revendication 11, **caractérisé en ce qu'**une partie (26) du dispositif de mémoire et de traitement (17) est relié au poste chirurgical (10) par l'intermédiaire d'un dispositif de transmission de données (25).

13. Système selon une des revendications précédentes, **caractérisé en ce que** le dispositif de mémoire et de traitement (17) est relié à un dispositif d'entrée (28) placé à l'extérieur du poste chirurgical (10).
